# EUROPÄISCHE PATENTANMELDUNG

(11) **EP 4 787 635 A1**
(43) Veröffentlichungstag der Anmeldung: **05.08.2026**
(21) Anmeldenummer: 25154675.0
(22) Anmeldetag: 29.01.2025
(51) Int. Cl.: H01R 24/58, A61M 60/00, H01R 13/52, H01R 107/00

(54) **STECKVERBINDUNGSEINRICHTUNG**

(71) Anmelder: Berlin Heart GmbH, 12247 Berlin (DE)
(72) Erfinder: WANGE, Michael, 12247 Berlin (DE); SCHÄRFF, Eike, 12247 Berlin (DE)
(74) Vertreter: Pfenning, Meinig & Partner mbB

(57) **Zusammenfassung**

Diese Offenbarung beschreibt eine Steckverbindungseinrichtung (100) zum Herstellen einer lösbaren elektrischen Verbindung zwischen zwei Komponenten eines mechanischen Herzunterstützungssystems. Die Steckverbindungseinrichtung (100) umfasst ein Steckverbindungselement (110) sowie eine Mehrzahl elektrischer Leiter (140A-E). Das Steckverbindungselement (110) weist einen länglich ausgebildeten Verbindungsabschnitt (112) zum Herstellen der lösbaren elektrischen Verbindung und eine Mehrzahl von voneinander isolierten elektrischen Kontaktelementen (114A-E), die entlang des Verbindungsabschnitts angeordnet sind, auf. Ferner ist mindestens eine der Mehrzahl der elektrischen Kontaktelemente (114A-H) als redundant verbundenes Kontaktelement (114A-C) ausgeführt, wobei das redundant verbundene Kontaktelement (114A-C) an mindestens zwei voneinander beabstandeten Kontaktbereichen (114A.1,2, 114B.1,2, 114C.1,2) fest und elektrisch leitend mit demselben der Mehrzahl elektrischer Leiter (140A-H) verbunden ist und/oder das redundant verbundene Kontaktelement (114A-C) an mindestens zwei voneinander beabstandeten Kontaktbereichen (114A.1,2, 114B.1,2, 114C.1,2) fest und elektrisch leitend mit je einem anderen der Mehrzahl elektrischer Leiter (140A-H) verbunden ist.

## Beschreibung

Für einen Betrieb eines implantierten, aktiven medizinischen Gerätes muss das medizinische Gerät mit elektrischer Energie wie auch häufig mit Daten wie beispielsweise Steuersignalen versorgt werden. Die Versorgung erfolgt dabei häufig kabelgebunden. Bei einem solchen implantierten, aktiven medizinischen Gerät kann es sich beispielsweise um einen Herzschrittmacher, einen implantierbaren Defibrillator oder auch eine implantierbare Blutpumpe für eine mechanische Unterstützung des Herzens handeln.

Um ein wiederholtes Trennen und Verbinden des medizinischen Gerätes von Steuereinheit und/oder Energiequelle bspw. für einen Austausch zu ermöglichen, weisen entsprechende Kabel meist einen lösbaren Steckverbinder auf. Diese Steckverbinder müssen dabei so gestaltet sein, dass sie eine hohe Sicherheit gegenüber einer Leitungsunterbrechung aufweisen.

Um den hohen Sicherheitsstandards zu genügen, werden Kontaktelemente zur Herstellung einer elektrischen Verbindung zwischen zwei Steckverbindungselementen des Steckverbinders häufig redundant ausgelegt. Dies kann jedoch nachteilig für die Größe des resultierenden Steckverbinders sein.

Die Aufgabe der vorliegenden Offenbarung ist es daher, ein verbessertes Konzept für einen Steckverbinder aufzuzeigen. Dieses verbesserte Konzept wird realisiert durch die Steckverbindungseinrichtung nach Anspruch 1 sowie durch das männliche Steckverbindungselement nach Anspruch 14. Optionale Merkmale werden in den abhängigen Ansprüchen beschrieben.

Die Steckverbindungseinrichtung dient zum Herstellen einer lösbaren elektrischen Verbindung zwischen zwei Komponenten eines mechanischen Herzunterstützungssystems und umfasst ein Steckverbindungselement und eine Mehrzahl elektrischer Leiter. Das Steckverbindungselement weist einen länglich ausgebildeten Verbindungsabschnitt zum Herstellen der lösbaren elektrischen Verbindung und eine Mehrzahl von voneinander isolierten elektrischen Kontaktelementen, die entlang des Verbindungsabschnitts angeordnet sind, auf. Zudem ist mindestens eines der Mehrzahl der elektrischen Kontaktelemente als redundant verbundenes Kontaktelement ausgeführt, wobei das redundant verbundene Kontaktelement an mindestens zwei voneinander beabstandeten Kontaktbereichen fest und elektrisch leitend mit demselben der Mehrzahl elektrischer Leiter verbunden ist und/oder das redundant verbundene Kontaktelement an mindestens zwei voneinander beabstandeten Kontaktbereichen fest und elektrisch leitend mit je einem anderen der Mehrzahl elektrischer Leiter verbunden ist.

Bei Blutpumpen für die mechanische Herzunterstützung müssen über die Steckverbinder sowohl Leiter für Steuersignale als auch eine Vielzahl von Leitern für die Energieversorgung der Blutpumpe miteinander verbunden werden. Ferner werden zum Herstellen einer Ausfallsicherheit redundante Kontaktelemente für mehrere der Leiter bereitgestellt. Hierdurch ergibt sich bei in Reihe entlang des Verbindungsabschnitts angeordneten Kontaktelementen ein sehr langer Steckverbinder. Gerade bei zur Implantation vorgesehenen Steckverbindern ist dies jedoch kontraproduktiv, da diese großen Steckverbinder für bestimmte Zielgruppen, wie beispielsweise Kindern, nicht mehr verwendet werden können. Die Erfinder haben erkannt, dass zum Herstellen einer Ausfallsicherheit redundante Kontaktelemente eine untergeordnete Rolle spielen, da diese meist großflächig ausgebildet sind und ein Ausfall einer Leitfähigkeit der Kontaktelemente unwahrscheinlich ist. Eine größere Anfälligkeit gegenüber Verschleiß ist bei den Leitern selbst zu beobachten. Zudem kann es auch an den Kontaktbereichen zwischen Kontaktelement und elektrischem Leiter zu Verschleiß kommen. Die beschriebene Lösung umfasst daher ein redundant verbundenes Kontaktelement, das mindestens zwei voneinander beabstandete Kontaktbereiche aufweist, über die das redundant verbundene Kontaktelement mit der Mehrzahl elektrischer Leiter verbunden ist. Ferner sind die mindestens zwei voneinander beabstandeten Kontaktbereiche mit demselben der Mehrzahl elektrischer Leiter verbunden und/oder die mindestens zwei der voneinander beabstandeten Kontaktbereiche mit je einem anderen der Mehrzahl elektrischer Leiter verbunden. Durch die mehrfache Kontaktierung kann die Anzahl der Kontaktelemente reduziert werden, was kleinere Steckverbinder ermöglicht.

Unter dem Begriff Steckverbinder wird ein System aus zwei ineinander steckbare Komponenten verstanden, das einem mechanischen und elektrischen Trennen und Verbinden von elektrischen Leitungen dient. Der Begriff Steckverbindungselement bezeichnet dabei jeweils eine dieser Komponenten. Wie in der Fachliteratur üblich, wird auch in dieser Offenbarung bei den Steckverbindungselementen zwischen männlichen und weiblichen Steckverbindungselementen unterschieden.

Eine Längsrichtung des Verbindungsabschnitts kann entlang einer Einsteckrichtung, entlang der das Steckverbindungselement in ein anderes hierfür vorgesehenes Steckverbindungselement zum Herstellen der lösbaren elektrischen Verbindung eingesteckt wird, ausgerichtet sein. Ferner kann die Mehrzahl der elektrischen Kontaktelemente hintereinander entlang einer Längsrichtung des Verbindungsabschnitts angeordnet sein. Weiterhin können die Kontaktelemente als Kontaktflächen ausgebildet sein.

Ferner kann jede der Kontaktelemente eine erste Oberfläche zur Herstellung der lösbaren elektrischen Verbindung und eine davon verschiedene zweite Oberfläche aufweisen. Zudem können die mindestens zwei voneinander beabstandeten Kontaktbereiche auf der zweiten Oberfläche angeordnet sein.

Weiterhin können entlang des Verbindungsabschnitts, vorzugsweise zwischen den elektrischen Kontaktelementen, eine oder mehrere Dichtlippen angeordnet sein.

Außerdem kann die Steckverbindungseinrichtung ein Kabel umfassen und die Mehrzahl der elektrischen Leiter die Adern des Kabels sein. Vorzugsweise sind die elektrischen Leiter in diesem Fall innerhalb des Kabels voneinander elektrisch isoliert.

Weiterhin kann es sich bei dem Steckverbindungselement um ein männliches Steckverbindungselement handelt. Bei dem männlichen Steckverbindungselement kann es sich folglich um einen Stecker, der zur Anordnung an einem Kabelende ausgebildet ist, oder einen Einbaustecker, der für einen Einbau in ein Gerätegehäuse ausgebildet ist, handeln.

Alternativ hierzu kann es sich bei dem Steckverbindungselement auch um ein weibliches Steckverbindungselement handelt. Bei dem weiblichen Steckverbindungselement kann es sich folglich um eine Kupplung, die zur Anordnung an einem Kabelende ausgebildet ist, oder um eine Buchse, die für einen Einbau in ein Gerätegehäuse ausgebildet ist, handeln.

Handelt es sich bei dem Steckverbindungselement um ein weibliches Steckverbindungselement, können die Kontaktelemente jeweils als Federkontakt, vorzugsweise als Ringfederkontakte, ausgebildet sein.

Weiterhin können die Kontaktelemente jeweils den Verbindungsabschnitt bzw. ein Inneres des Verbindungsabschnitts zumindest teilweise, vorzugsweise vollständig, umlaufen, bzw. einen offenen oder einen geschlossenen Ring bilden.

Zudem können die Kontaktbereiche des redundant verbundenen Kontaktelements entlang eines Umfangs des Kontaktelementes verteilt angeordnet seien und um mindestens 30°, vorzugsweise mindestens 90°, 120° oder 180°, voneinander beabstandet seien.

Die Mehrzahl elektrischer Leiter können ferner insgesamt acht Leiter umfassen und die Mehrzahl der Kontaktelemente insgesamt fünf Kontaktelemente umfassen, von denen drei, vorzugsweise genau drei, redundant verbunden sind, wobei für jedes der redundant verbundenen Kontaktelemente gilt, dass deren mindestens zwei voneinander beabstandeten Kontaktbereiche mit je einem anderen der Mehrzahl elektrischer Leiter verbunden sind.

Außerdem kann die Steckverbindungseinrichtung so ausgebildet sein, dass die Steckverbindungseinrichtung zumindest teilweise implantierbar ist. Vorzugsweise ist hierbei das Steckverbindungselement und ein an das Steckverbindungselement angrenzender Abschnitt der Mehrzahl elektrischer Leiter implantierbar.

Die Steckverbindungseinrichtung kann ferner Teil eines Steckverbindungssystems sein. Solch ein Steckverbindungssystem umfasst eine erste Steckverbindungseinrichtung nach einem der vorhergehend beschriebenen Ausführungsformen, wobei das Steckverbindungselement der ersten Steckverbindungseinrichtung ein erstes Steckverbindungselement ist. Zudem umfasst das Systemverbindungssystem ein zweites Steckverbindungselement, wobei das zweite Steckverbindungselement einen länglich ausgebildeten Verbindungsabschnitt zum Herstellen der lösbaren elektrischen Verbindung und eine Mehrzahl von voneinander isolierten elektrischen Kontaktelementen, die entlang des Verbindungsabschnitts angeordnet sind aufweist. Ferner sind das erste Steckverbindungselement und das zweite Steckverbindungselement zum lösbaren Herstellen der elektrischen Verbindung ineinander einführbar, wodurch ein paarweiser elektrischer Kontakt zwischen den Kontaktelementen des ersten Steckverbindungselements und den Kontaktelementen des zweiten Steckverbindungselements herstellbar ist.

Optional kann dabei auch die zweite Steckverbindungseinrichtung nach einem der vorhergehend beschriebenen Ausführungsformen der Steckverbindungseinrichtung ausgeführt sein, wobei in diesem Fall deren Steckverbindungselement das zweite Steckverbindungselement ausbildet.

Die Verbindungseinrichtung kann weiterhin Bestandteil eines mechanischen Herzunterstützungssystems sein. Solch ein Herzunterstützungssystem umfasst eine erste Komponente, eine zweite Komponente und ein Steckverbindungssystem entsprechend einer der zuvor beschriebenen Ausführungsformen, wobei eine lösbare elektrische Verbindung zwischen der ersten Komponente und der zweiten Komponente mittels des Steckverbindungssystems herstellbar ist.

Bei der ersten Komponente kann es sich um eine Herzpumpe handeln. Bei der zweiten Komponente kann es sich um eine Steuer- und/oder Energieversorgungseinrichtung für die Herzpumpe handeln.

Unabhängig von der Idee eines redundant verbundenen Kontaktelements haben die Erfinder ebenfalls erkannt, dass es Verbesserungsbedarf bei Dichtelementen, die einem Abdichten des Steckverbinders dienen, gibt. Zu einer Verbesserung kann das im Folgenden beschriebene männliche Steckverbindungselement zum Herstellen einer lösbaren elektrischen Verbindung zwischen zwei Komponenten eines mechanischen Herzunterstützungssystems beitragen.

Das männliche Steckverbindungselement umfasst einen länglich ausgebildeten Verbindungsabschnitt zum Herstellen der lösbaren elektrischen Verbindung und eine Mehrzahl von voneinander isolierten elektrischen Kontaktelementen, die entlang des Verbindungsabschnitts angeordnet sind. Ferner umfasst das männliche Steckverbindungselement entlang des Verbindungsabschnitts, vorzugsweise zwischen den elektrischen Kontaktelementen, eine oder mehrere Dichtelemente.

Die Erfinder haben erkannt, dass Dichtelemente häufig in einem buchsenseitigen, d.h. einem weiblichen, Steckverbindungselements angeordnet sind und damit Teil einer Komponente des mechanischen Herzunterstützungssystems wie beispielsweise der Blutpumpe sind. Die Dichtelemente unterliegen jedoch einem Verschleiß und können daher einen Austausch erfordern. Dies würde jedoch einen Austausch der gesamten Komponente erfordern, was einen hohen Aufwand bedeutet. Eine Verbindungsleitung, häufig auch als Driveline bezeichnet, zwischen Blutpumpe und Steuer- und/oder Energieeinheit wird jedoch in der Regel regelmäßig ausgetauscht. Das Anordnen der Dichtelemente am männlichen Steckverbindungselement hat damit den Vorteil, dass bei einem Austausch der Driveline auch die Dichtelemente ausgetauscht werden können, ohne dass die Komponente selbst ausgetauscht werden muss.

Bei den Dichtelementen kann es sich um Dichtlippen handeln. Ferner kann es sich bei den einen oder mehreren Dichtelementen um genau zwei oder genau drei Dichtelemente handeln, die zwischen zwei benachbarten Kontaktelementen angeordnet sind.

Eine Längsrichtung des Verbindungsabschnitts kann entlang einer Einsteckrichtung, entlang der das männliche Steckverbindungselement in ein entsprechendes weibliches Steckverbindungselement zum Herstellen der lösbaren elektrischen Verbindung eingesteckt wird, ausgerichtet sein. Ferner kann die Mehrzahl der elektrischen Kontaktelemente hintereinander entlang einer Längsrichtung des Verbindungsabschnitts angeordnet sein.

Weiterhin kann das männliche Steckverbindungselement Teil eines Steckverbindungssystems sein. Dieses Steckverbindungssystem umfasst das männliche Steckverbindungselement entsprechend einer der zuvor beschriebenen Ausführungsformen und ein weibliches Steckverbindungselement. Das weibliche Steckverbindungselement weist einen länglich ausgebildeten Verbindungsabschnitt zum Herstellen der lösbaren elektrischen Verbindung sowie eine Mehrzahl von voneinander isolierten elektrischen Kontaktelementen, die entlang des Verbindungsabschnitts angeordnet sind, auf. Ferner ist der Verbindungsabschnitt des männlichen Steckverbindungselements zum lösbaren Herstellen der elektrischen Verbindung in den Verbindungsabschnitt des weiblichen Steckverbindungselements einführbar, wodurch ein paarweiser elektrischer Kontakt zwischen den Kontaktelementen des männlichen Steckverbindungselements und den Kontaktelementen des weiblichen Steckverbindungselements herstellbar ist.

Die Idee des beschriebenen männlichen Steckverbindungselements ist unabhängig von der zuvor beschriebenen Idee der redundant verbundenen elektrischen Kontaktelemente. Beide Ideen können jedoch miteinander in einer Steckverbindungseinrichtung kombiniert werden.

Im Folgenden werden anhand der Figuren weitere Ausführungsbeispiele der Steckverbindungseinrichtung sowie des männlichen Steckverbindungselements entsprechend dieser Offenbarung beschrieben.

Die Figuren zeigen:
- Fig. 1: eine Seitenansicht einer Steckverbindungseinrichtung zum Herstellen einer lösbaren elektrischen Verbindung zwischen zwei Komponenten eines mechanischen Herzunterstützungssystems;
- Fig. 2: eine Querschnittsansicht der in Fig. 1 gezeigten Steckverbindungseinrichtung;
- Fig. 3: eine Querschnittsansicht eines zum Fig. 1 gezeigten alternativen Ausführungsbeispiels der Steckverbindungseinrichtung;
- Fig. 4: eine schematische Darstellung zur Anordnung von Kontaktbereichen um einen Umfang der in Fig. 1 gezeigten Steckverbindungseinrichtung;
- Fig. 5: eine Seitenansicht einer Steckverbindungseinrichtung mit einem weiblichen Steckverbindungselement zum Herstellen einer lösbaren elektrischen Verbindung zwischen zwei Komponenten eines mechanischen Herzunterstützungssystems;
- Fig. 6: eine Querschnittsansicht eines vorderen Teils der in Fig. 5 gezeigten Steckverbindungseinrichtung;
- Fig. 7: ein mechanisches Herzunterstützungssystem 400, das die in Fign. 1 und 5 gezeigten Steckverbindungseinrichtungen umfasst;
- Fig. 8: ein männliches Steckverbindungselement zum Herstellen einer lösbaren elektrischen Verbindung zwischen zwei Komponenten eines mechanischen Herzunterstützungssystems, das zwischen Dichtelemente zwischen benachbarten Kontaktelementen aufweist; und
- Fig. 9: einen Steckverbindungselement 910, dass eine Alternative des in Fig. 5 gezeigten Steckverbindungselements 310 darstellt.

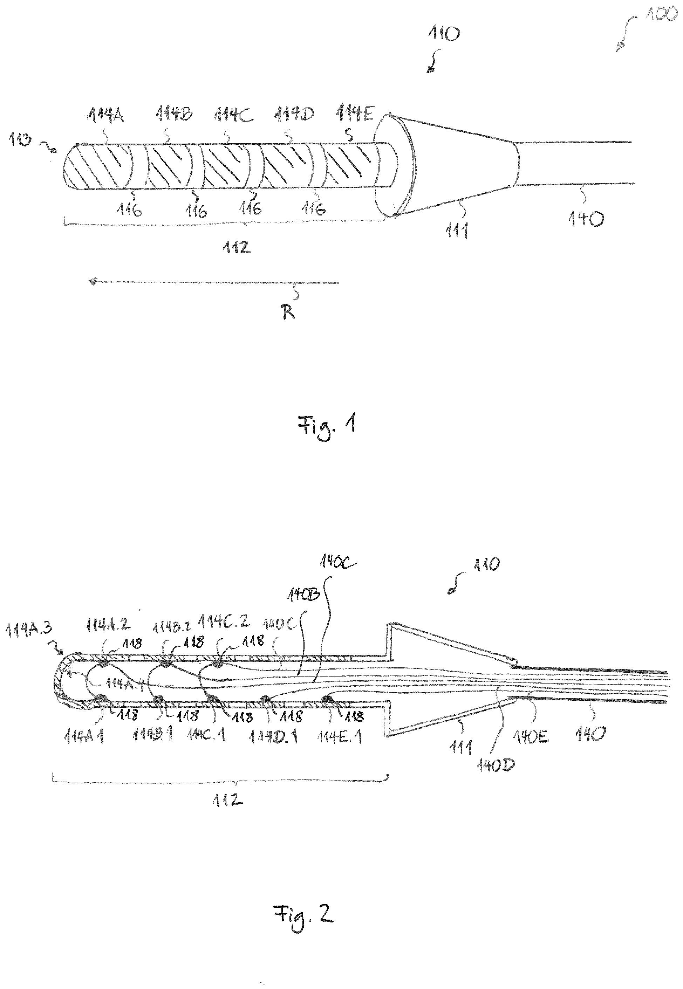

Fig. 1 zeigt eine Seitenansicht einer Steckverbindungseinrichtung 100 zum Herstellen einer lösbaren elektrischen Verbindung zwischen zwei Komponenten eines mechanischen Herzunterstützungssystems.

Die Steckverbindungseinrichtung 100 umfasst ein Steckverbindungselement 110 sowie eine Mehrzahl elektrischer Leiter.

In dem in Fig. 1 gezeigten Ausführungsbeispiel der Steckverbindungseinrichtung 100 handelt es sich bei dem Steckverbindungselement 110 um ein männliches Steckverbindungselement in Form eines Steckers. Ferner handelt es sich bei der Mehrzahl elektrischer Leiter um Adern eines Kabels 140.

Das Steckverbindungselement 110 weist einen Knickschutz 111, einen länglich ausgebildeten Verbindungsabschnitt 112 zum Herstellen der lösbaren elektrischen Verbindung auf sowie eine Mehrzahl von voneinander isolierten elektrischen Kontaktelementen 114A-E, die entlang des Verbindungsabschnitts 112 angeordnet sind und in den Figuren straffiert dargestellt sind. Eine Einführungsrichtung R des Steckers 110 in ein entsprechendes weibliches Steckverbindungselement entspricht dabei einer Längsrichtung des Verbindungsabschnitts 112.

Im Beispiel der Fig. 1 ist der Verbindungsabschnitt 112 in Form eines Stabs 113 ausgebildet, entlang dessen Längsrichtung die Kontaktelemente 114A-E in Reihe hintereinander angeordnet sind. Die Kontaktelemente 114A-E sind dabei als Ringe ausgebildet und umlaufen den Stab 113 in dem gezeigten Beispiel vollumfänglich. Zur elektrischen Isolierung der Kontaktelemente 114A-E sind jeweils zwei benachbarte der Mehrzahl elektrischer Kontaktelemente 114A-E mittels elektrisch isolierenden Bereichen 116 voneinander getrennt. Diese elektrisch isolierenden Bereiche können beispielsweise durch Kunststoffelemente gebildet werden. Die Kontaktelemente 114A-E sind elektrisch leitend und können beispielsweise aus Kupfer oder Gold hergestellt sein.

Ferner sind drei der Mehrzahl elektrischer Kontaktelemente 114A-E als redundant verbundene Kontaktelemente ausgeführt, wobei jedes der redundant verbundenen Kontaktelemente an zwei voneinander beabstandeten Kontaktbereichen fest und elektrisch leitend mit einem der Mehrzahl elektrischer Leiter verbunden sind. Für eine detailliertere Beschreibung der redundant verbundenen Kontaktelemente wird im Folgenden auf Fig. 2 Bezug genommen.

Fig. 2 zeigt eine Querschnittsansicht der in Fig. 1 gezeigten Steckverbindungseinrichtung 100. Der in Fig. 2 gezeigte Querschnitt entspricht dabei einem Querschnitt durch den Stecker 100 entlang einer Längsachse des Stabs 113 und zeigt eine schematische Innenansicht des Steckers 100.

In der Querschnittsansicht der Fig. 2 sind die Mehrzahl elektrischer Leiter innerhalb des Kabels 140 zu erkennen. Die Mehrzahl elektrischer Leiter umfasst insgesamt fünf elektrische Leiter 140A-E.

In Fig. 2 ist ebenfalls zu erkennen, dass jeder der Kontaktelemente 114A-E eine erste, d.h. eine äußere Oberfläche zur Herstellung der lösbaren elektrischen Verbindung und eine davon abgewandte zweite, d.h. innere Oberfläche aufweist. Dies ist in Fig. 2 am Beispiel des Kontaktelements 114A gezeigt, dessen äußere Oberfläche mit dem Bezugszeichen 114A.3 und dessen innere Oberfläche mit dem Bezugszeichen 114A.4 versehen ist.

Ferner sind in Fig. 2 die Kontaktbereiche für jede der Kontaktelemente 114A-E zu erkennen, die auf der inneren Oberfläche der jeweiligen Kontaktelemente 114A-E angeordnet sind. Die Kontaktelemente 114D und 114E umfassen dabei insgesamt nur jeweils einen Kontaktbereich 114D.1 und 114E.1, an denen die Kontaktelemente 114D und 114E mit dem elektrischen Leiter 140D bzw. 140E elektrisch verbunden sind. Die Kontaktelemente 114A-C hingegen weisen jeweils zwei Kontaktbereiche 114A.1,2, 114B.1,2 und 114C.1,2 auf, wobei die Kontaktbereiche eines jeweiligen Kontaktelementes um 180° entlang des Umfang des gegebenen Kontaktelements zueinander beabstandet sind. Bei den Kontaktelementen 114A-C handelt es sich daher um redundant verbundene Kontaktelemente. An den Kontaktbereichen ist ein Kontaktelement jeweils mit einem der Mehrzahl elektrischer Leiter, beispielsweise wie im Beispiel gezeigt über einen Anschluss 118, elektrisch verbunden. Der Anschluss 118 kann beispielsweise über eine Schweißverbindung realisiert sein. Es sind aber auch andere Fügeverbindungen möglich. Für jedes der redundanten Kontaktelement gilt in diesem Ausführungsbeispiel, dass die Kontaktbereiche eines Kontaktelements mit demselben Leiter verbunden. In dieser Ausführungsform sind insgesamt weniger Leiter notwendig, als wenn zusätzliche Kontaktelemente einzeln kontaktiert werden.

Bei dem in Fig. 2 gezeigten Beispiel geht es darum, eine Redundanz in der Verbindung zwischen den elektrischen Leitern 140A-C und den Kontaktelementen 114A-C herzustellen. Aus diesem Grund ist der elektrische Leiter 140A über den Kontaktbereich 114A.1 und über den Kontaktbereich 114A.2 mit dem Kontaktelement 114A verbunden. Sollte eine der Anschlüsse durch beispielsweise Korrosion versagen, so ist eine elektrische Verbindung zwischen dem Kontaktelement 114A und dem elektrischen Leiter 140 trotzdem über den jeweils anderen verbleibenden Kontaktbereich gegeben. Gleiches gilt für die elektrische Verbindung zwischen dem Kontaktelement 114B und dem elektrischen Leiter 140B sowie dem Kontaktelement 114C und dem elektrischen Leider 140C.

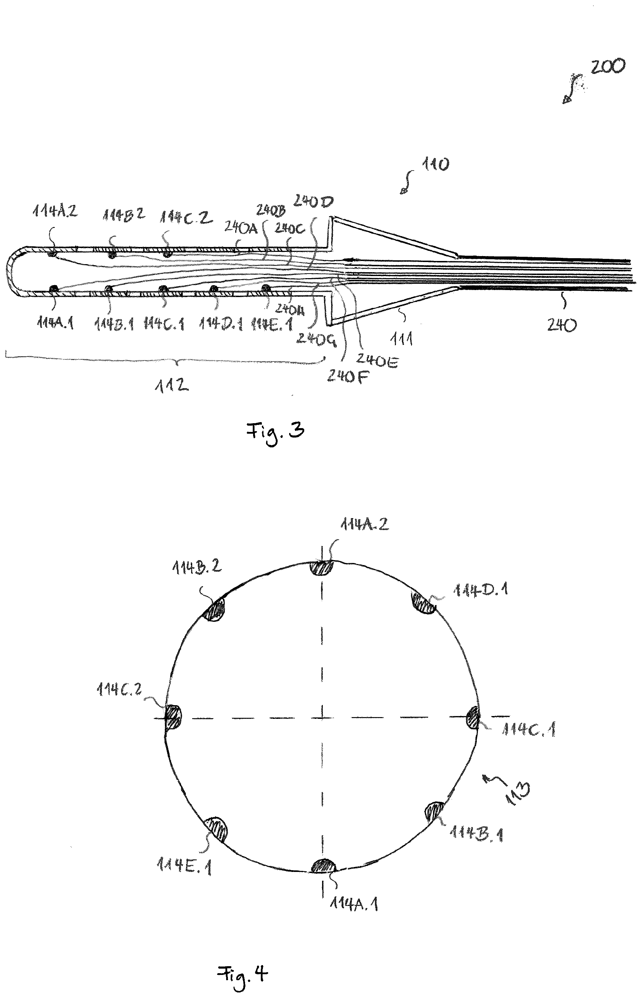

Neben der Redundanz der Kontaktbereiche kann auch eine Redundanz bei den elektrischen Leitern selbst gewünscht sein. Wie solch eine Redundanz in Verbindung mit dem redundant verbundenen Kontaktbereichen erreicht werden kann, wird im Folgenden mit Bezug auf Fig. 3 beschrieben.

Fig. 3 zeigt eine Querschnittsansicht eines zum Fig. 1 gezeigten alternativen Ausführungsbeispiels 200 der Steckverbindungseinrichtung.

Die Steckverbindungseinrichtung 200 umfasst ebenfalls das Steckverbindungselement 110, das bereits in Hinblick auf Steckverbindungseinrichtung 100 beschrieben wurde. Statt des Kabels 140 mit insgesamt fünf elektrischen Leitern umfasst die Steckverbindungseinrichtung 200 jedoch ein Kabel 240, das insgesamt acht Adern 240A-H aufweist.

In dem in Fig. 3 gezeigten Beispiel sind die elektrischen Leiter 240C und 240D, die Leiter 240B und 240E und die Leiter 240A und 240F jeweils redundant ausgelegt und übertragen identische Signale. Leiter 240A und Leiter 240F sind daher über die Kontaktbereiche 114A.1 und 114A.2 beide mit dem Kontaktelement 114A elektrisch leitend verbunden. Gleiches gilt auch für die Leiter 240B und 240E, die beide über die Kontaktbereiche 114B.1,2 mit dem Kontaktelement 114B elektrisch leitend verbunden sind, sowie die Leiter 240C und 240D, die beide über die Kontaktbereiche 114C.1,2 mit dem Kontaktelement 114C verbunden. Die elektrischen Leiter 240G und 240H sind hingegen jedoch nicht redundant ausgebildet und auch nur über einen Kontaktbereich mit dem Kontaktelement 114D bzw. 114E elektrisch verbunden. In diesem Ausführungsbeispiel sind die drei Kontaktelemente in voneinander beabstandeten Kontaktbereichen mit voneinander getrennten Leitern kontaktiert. Zwar werden in diesem Ausführungsbeispiel mehr Leiter benötigt als im vorhergehenden Ausführungsbeispiel, aber die Kontaktierung einzelner Kontaktelemente mit mehr als einem Leiter (in zwei voneinander getrennten Kontaktbereichen) erhöht die Ausfallsicherheit gegenüber Leiterbrüchen oder -ausfällen und Anschlussausfällen. Die Leiter können dabei über ihre gesamte Länge voneinander elektrisch isoliert sein, oder die ein Kontaktelement kontaktierenden Leiter können miteinander verbunden, beispielsweise verdreht oder verwoben werden.

Herzpumpen müssen konstant mit elektrischer Energie versorgt werden. Eine Unterbrechung der Energieversorgung wäre für einen Patienten lebensbedrohlich. In dem in Fig. 3 gezeigten Beispiel können die redundanten Leiterpaare 240C und 240D, 240B und 240E sowie 240A und 240F beispielsweise für einen Betrieb eines dreipoligen Motors einer Herzpumpe jeweils eine redundante Verbindung zu einem U-, V- und W-Pol ermöglichen. Die nicht redundanten elektrischen Leiter 240G und 240H können für eine Übertragung von Steuersignalen verwendet werden, die nicht zwingend einer Redundanz bedarf. Durch die Verwendung von redundant verbundenen Kontaktelementen kann die Anzahl der Kontaktelemente für die elektrische Übertragung in diesem Beispiel von acht auf fünf reduziert werden, was einer Längenreduzierung von 30% entspricht.

In der Darstellung von Fig. 2 und 3 liegen die Kontaktbereiche 114A.2, 114B.2 und 114C.2 sowie die Kontaktbereiche 114A.1, 114B.1, 114C.1, 114D.1 und 114E.1 in einer Querschnittsebene entlang der Längsachse des Stabes 113. Vorteilhaft kann es jedoch sein, die Kontaktbereiche entlang des Umfangs des Stabs 113 zu versetzen. Solche eine vorteilhafte Anordnung wird im Folgenden mit Bezugnahme auf Fig. 4 beschrieben.

Fig. 4 zeigt eine schematische Darstellung zur Anordnung von Kontaktbereichen um einen Umfang der in Fig. 1 gezeigten Steckverbindungseinrichtung.

Die Darstellung zeigt eine vorteilhafte Anordnung der Kontaktbereiche innerhalb des Stabs 113, wenn dieser mit Blickrichtung entlang dessen Längsachse betrachtet wird. Zu sehen ist der kreisrunde Umfang des Stabs 113 und die Anordnung von insgesamt acht Kontaktbereichen, die den in Fig. 2 und 3 gezeigten Kontaktbereichen entsprechen. Die Kontaktbereiche 114A.1, 114A.2 sowie 114B.1, 114B.2 und 114C.1, 114C.1, die zu einem redundant verbundenen Kontaktelement gehören, sind dabei entlang des Umfang des Stabs 113 auf gegenüberliegenden Seiten angeordnet und damit mit einem Winkel von 180° beabstandet. Hierdurch kann das Risiko minimiert werden, dass eine Korrosion eines Kontaktbereichs den anderen Kontaktbereich des gleichen Kontaktelements ebenfalls betrifft und außerdem eine optimale elektrische Lastverteilung über das jeweilige Kontaktelement erreicht werden.

Auch die Kontaktbereiche 114D.1 und 114E.1 wurden entlang des Umfangs des Stabs 113 auf gegenüberliegenden Seiten angeordnet. Ferner sind jeweils gegenüberliegende Kontaktbereichspaare eines Kontaktelements mit einem Bogenmaß von π/4 entlang des Umfangs des Stabs 113 zum jeweils nächsten Kontaktbereich der anderen Kontaktelemente beabstandet. Hierdurch kann das Innere des Stabes 113 besser für eine Führung der Adern des Kabels 140 bzw. 240 verwendet werden.

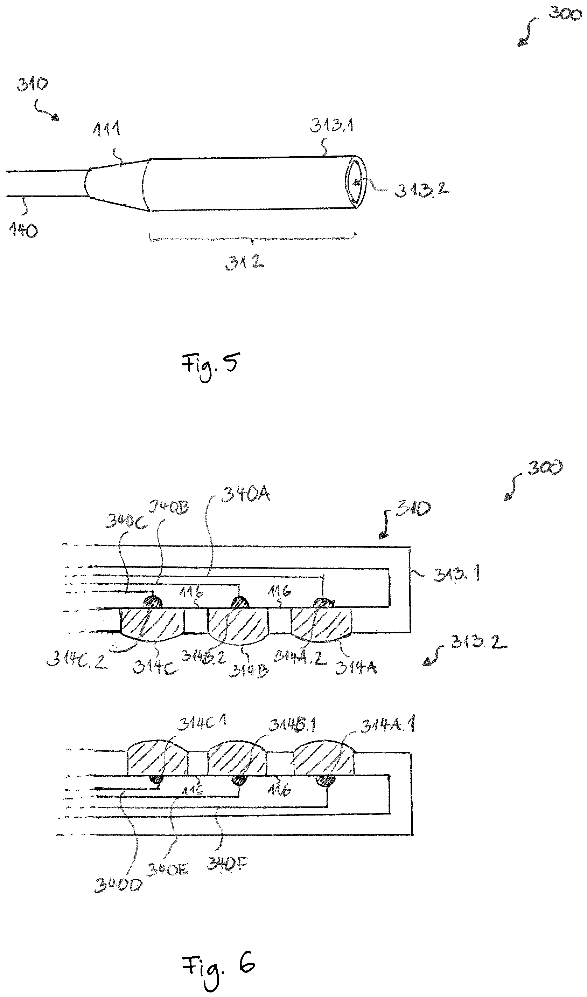

Anstatt eines männlichen Steckverbindungselements kann das in dieser Offenbarung beschriebene Konzept auch für die Realisierung eines weiblichen Steckverbindungselementes benutzt werden. Ein solches Ausführungsbeispiel wird im Folgenden mit Bezug auf Fign. 5 und 6 beschrieben.

Fig. 5 zeigt eine Seitenansicht einer Steckverbindungseinrichtung 300 mit einem weiblichen Steckverbindungselement 310 zum Herstellen einer lösbaren elektrischen Verbindung zwischen zwei Komponenten eines mechanischen Herzunterstützungssystems. Fig. 6 zeigt eine Querschnittsansicht eines vorderen Teils der in Fig. 5 gezeigten Steckverbindungseinrichtung 300.

Die Steckverbindungseinrichtung 300 umfasst das Steckverbindungselement 310, das beispielsweise mit dem Steckverbindungselement 110 aus Fig. 1 zur Herstellung einer elektrischen Verbindung zusammen wirken kann. Das Steckverbindungselement 310 weist ebenfalls den Knickschutz 111 auf. Ferner weist das Steckverbindungselement 310 ein zylindrisches Gehäuse 313.1 mit einer Gehäuseöffnung 313.2 auf, das einem Verbindungsabschnitt 312 entspricht. Ein vorderer, unmittelbar an der Gehäuseöffnung 313.2 angrenzender Teil des Verbindungsabschnitts 312 ist im Querschnitt in Fig. 6 gezeigt.

Fig. 6 zeigt drei im Inneren des zylindrischen Gehäuses 313.1 angeordnete Kontaktelemente 314A-C. Die Kontaktelemente 314A-C sind als Ringkontakte ausgebildet und umlaufen jeweils das Innere des zylindrischen Gehäuses 313.1. Die Kontaktelemente 314A-C sind ferner hintereinander entlang einer Längsachse des zylindrischen Gehäuses 313.1 angeordnet, wobei jeweils benachbarte Kontaktelemente durch elektrisch isolierende Bereiche voneinander beabstandet sind.

Ferner umfasst die Steckverbindungseinrichtung 300 auch eine Mehrzahl elektrischer Leiter, von denen in Fign. 5 und 6 nur sechs Leiter 340A-F gezeigt sind. Über Leiterpaare 340A,F, 340B,E und 340C,D werden dabei jeweils die gleichen Signale übertragen.

Weiterhin handelt es sich bei allen in Fig. 6 gezeigten Kontaktelement 314A-C um redundant verbundene Kontaktelemente, die jeweils zwei Kontaktbereiche 314A.1,2, 314B.1,2 und 314C.1,2 aufweisen. Das redundante Leiterpaar 340A,F ist dabei über die Kontaktbereiche 314A.1,2 mit dem Kontaktelement 314A verbunden, das redundante Leiterpaar 340B,E dabei über die Kontaktbereiche 314B.1,2 mit dem Kontaktelement 314B und das redundante Leiterpaar 340C,D über die Kontaktbereiche 314C.1,2 mit dem Kontaktelement 314C.

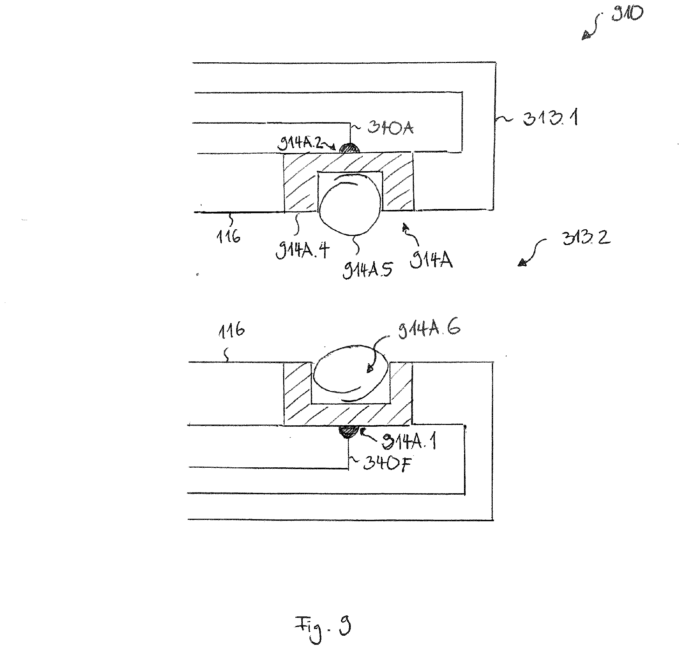

In der in Fign. 5 und 6 gezeigten Ausführungsform der Steckverbindungseinrichtung 300 sind die Kontaktelemente 314A-C als statische Ringe ausgebildet. Alternativ hierzu können die Kontaktelemente auch als Ringfederkontakteausgebildet sein. Dies wird unter Bezugnahme auf Fig. 9 im Folgenden detaillierter beschrieben.

Fig. 9 zeigt einen Steckverbindungselement 910, dass eine Alternative des in Fig. 5 gezeigten Steckverbindungselements 310 darstellt. Das Steckverbindungselement 910 ist dabei in einer Querschnittsansicht gezeigt, bei der nur ein unmittelbar an der Gehäuseöffnung 313.2 angeordnetes Kontaktelement 914A gezeigt ist.

Das Steckverbindungselement 910 ist identisch zu dem Steckverbindungselement 310 mit Ausnahme von dem Kontaktelement 914A. Bei dem Kontaktelement 914A handelt es sich um einen sogenannten Federringkontakt.

Das Kontaktelement 914A umfasst ein ringförmiges Basiselement 914A.4, das das Innere des zylindrischen Gehäuses 313.1 vollumfänglich umläuft. Das ringförmige Basiselement 914A.4 weist ferner in einer in das Gehäuseinnere gerichteten Oberfläche eine Nut 914A.6 auf, die das ringförmige Basiselement 914A.4 ebenfalls vollständig umläuft. In der Nut 914A.6 ist weiterhin eine vollumfänglich verlaufende Feder 914A.5 angeordnet. Die Feder 914A.5 steht über die nach innen gerichtete Oberfläche des ringförmigen Basiselements 914A.4 hinaus. Wird ein entsprechender Stecker in das weibliche Steckverbindungselement 910 eingeführt, so wird die Feder gegen Kontaktelemente des Steckers gedrückt, was zu einer besonders sicheren elektrischen Kontakt zwischen den Steckverbindungselementen führen kann.

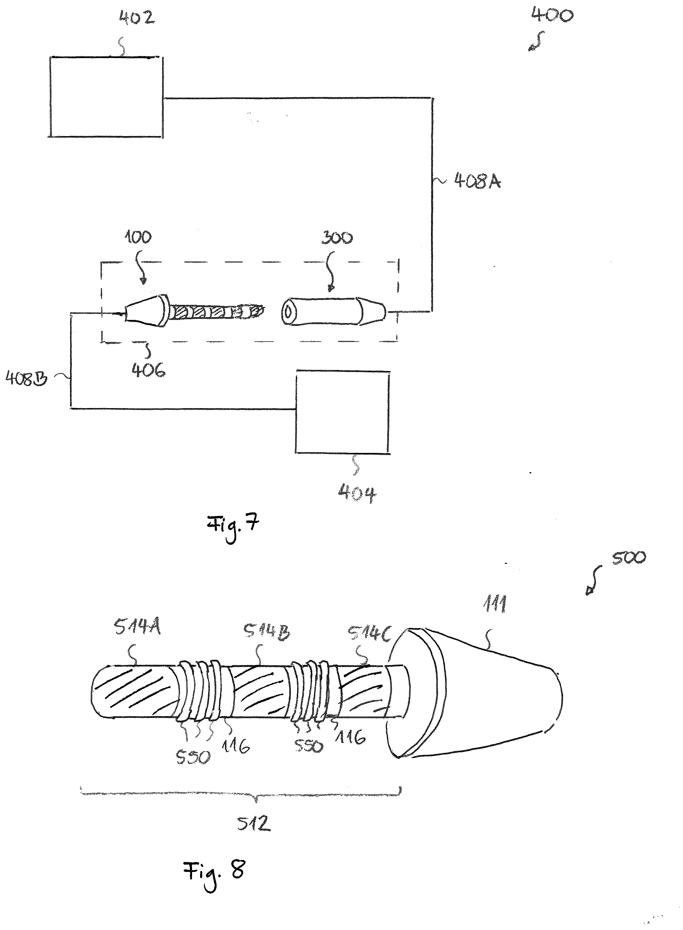

Wie bereits eingangs erläutert, können die in den Fign. 1-6 und 9 gezeigten Steckverbindungseinrichtungen für die Herstellung einer Verbindung zwischen zwei Komponenten eines mechanischen Herzunterstützungssystems verwendet werden. Solch ein mechanisches Herzunterstützungssystem wird im Folgenden mit Bezugnahme auf Fig. 7 beschrieben.

Fig. 7 zeigt ein mechanisches Herzunterstützungssystem 400, das die in Fign. 1 und 5 gezeigten Steckverbindungseinrichtungen umfasst.

Das in Fig. 7 gezeigte mechanische Herzunterstützungssystem 400 umfasst eine erste Komponente 402, bei der es sich um eine Blutpumpe handelt, und eine zweite Komponente 404, bei der es sich um eine Steuer- und Energieversorgungseinrichtung für die Blutpumpe 402 handelt. Eine Übertragung von Steuersignalen und Energie zwischen der ersten Komponente 402 und der zweiten Komponente 404 erfolgt über Kabel 408A und 408B, die jeweils mit einer der Komponenten elektrisch verbunden sind. Eine elektrische Verbindung zwischen den Kabeln 408A und 408B erfolgt über ein Steckverbindungssystem 406, das die Steckverbindungseinrichtung 100 und die Steckverbindungseinrichtung 300 umfasst. Über das Steckverbindungssystem 406 ist es möglich eine lösbare elektrische Verbindung zwischen der ersten Komponente 402 und der zweiten Komponente 404 herzustellen.

Die elektrische Verbindung über die Kabel 408A,B inkl. dem Steckverbindungssystem 406 wird auch als Driveline bezeichnet. Bei einer implantierten Blutpumpe 402 wird die Driveline teilweise mitimplantiert und tritt aus dem Körper eines Patienten durch die Haut aus. In Fällen, bei denen auch das Steckverbindungssystem implantiert werden soll, sind die jeweiligen Steckverbindungseinrichtungen entsprechend implantierbar ausgestaltet.

In dem in Fig. 7 gezeigten Steckverbindungssystem 406 umfassen beide Steckverbindungselemente redundant verbundene Kontaktelemente. Es ist jedoch auch möglich, dass nur einer der Steckverbindungselemente einen Verbindungsabschnitt mit einem oder mehreren redundant verbundenen Kontaktelementen aufweist. Auch ist es beispielsweise möglich, dass entweder das männliche Steckverbindungselement 100 anstatt als Stecker als Einbaustecker oder das weibliche Steckverbindungselement 300 anstatt als Kupplung als Buchse ausgebildet ist. Ferner ist es auch möglich, dass die Anzahl der Kontaktelemente pro Steckverbindungselement eine andere ist.

Wie eingangs bereits beschrieben, ist es allgemein üblich, dass Dichtelemente zum Abdichten der Steckverbindung gegen Partikel und Flüssigkeit im weiblichen Steckverbinder angeordnet sind. Dies ist jedoch nachteilig, wenn der weibliche Steckverbinder als Buchse einer der Komponenten ausgebildet ist und damit zum Austauschen der Dichtelemente die gesamte Komponente ausgetauscht werden muss. Auf die Lösung dieses Problems zielt eine andere Entwicklung dieser Offenbarung ab, die im Folgenden mit Bezugnahme auf Fig. 8 beschrieben werden soll.

Fig. 8 zeigt ein männliches Steckverbindungselement 500 zum Herstellen einer lösbaren elektrischen Verbindung zwischen zwei Komponenten eines mechanischen Herzunterstützungssystems, das Dichtelemente zwischen benachbarten Kontaktelementen aufweist.

Das männliche Steckverbindungselement 500 ist in Form eines Steckers ausgebildet und weist einen länglich ausgebildeten Verbindungsabschnitt 512 zum Herstellen der lösbaren elektrischen Verbindung auf und eine Mehrzahl von elektrischen Kontaktelementen 514A-C, die entlang des Verbindungsabschnitts 512 hintereinander angeordnet sind. Der Verbindungsabschnitt 512 ist dabei so ausgebildet, dass bei Einführen des männlichen Steckverbindungselements 500 in ein entsprechendes weibliches Steckverbindungselement ein paarweiser elektrischer Kontakt zwischen den Kontaktelementen des männlichen Steckverbindungselements 500 und Kontaktelementen des weiblichen Steckverbindungselements hergestellt wird.

Zwischen jeweils benachbarten Kontaktelementen, d.h. zwischen Kontaktelement 514A und 514B sowie zwischen 514B und 514C, befindet sich jeweils ein elektrisch isolierender Bereich 116, der die angrenzenden Kontaktelemente voneinander elektrisch isoliert. Zudem sind in jedem elektrisch isolierenden Bereich 116 jeweils drei Dichtelemente 550 nebeneinander angeordnet. Bei den Dichtelementen 550 handelt es sich in dem Beispiel der Fig. 8 um Dichtlippen, die die elektrisch isolierenden Bereiche 116 vollumfänglich umlaufen. Die Dichtlippen 550 sind ausgebildet, wenn der Stecker in das entsprechende weibliches Steckverbindungselement eingeführt ist, mit diesem zusammenzuwirken, um einem Eindringen von Partikeln und Fluiden in den Steckverbinder entgegenzuwirken.

In dem in Fig. 8 gezeigten Beispiel umfasst das männliche Steckverbindungselement 500 genau drei Kontaktelemente 514A-C. Dies können aber in anderen Ausführungsbeispielen auch mehr oder weniger Kontaktelemente sein. Ebenfalls sind im Beispiel der Fig. 8 immer drei Dichtelemente 550 nebeneinander angeordnet. Auch dies können in anderen Ausführungsformen mehr oder weniger Dichtelemente sein.

Die in Fig. 8 gezeigten Dichtlippen 550 können zusammen mit den in Zusammenhang mit Fign. 1-7, 9 beschriebenen redundant verbundenen Kontaktelementen verwendet werden. Dies kann vorteilhaft sein, um einen Verschließ der Kontaktelemente weiter zu verringern. Eine Verwendung von redundant verbundenen Kontaktelementen ist bei dem männlichen Steckverbindungselement 550 jedoch nicht zwingend erforderlich.

Zusammenfassend wird in dieser Offenbarung eine Steckverbindungseinrichtung (100) zum Herstellen einer lösbaren elektrischen Verbindung zwischen zwei Komponenten eines mechanischen Herzunterstützungssystems beschrieben. Die Steckverbindungseinrichtung (100) umfasst ein Steckverbindungselement (110) sowie eine Mehrzahl elektrischer Leiter (140A-E). Das Steckverbindungselement (110) weist einen länglich ausgebildeten Verbindungsabschnitt (112) zum Herstellen der lösbaren elektrischen Verbindung und eine Mehrzahl von voneinander isolierten elektrischen Kontaktelementen (114A-E), die entlang des Verbindungsabschnitts angeordnet sind, auf. Ferner ist mindestens eine der Mehrzahl der elektrischen Kontaktelemente (114A-H) als redundant verbundenes Kontaktelement (114A-C) ausgeführt, wobei das redundant verbundene Kontaktelement (114A-C) an mindestens zwei voneinander beabstandeten Kontaktbereichen (114A.1,2, 114B.1,2, 114C.1,2) fest und elektrisch leitend mit demselben der Mehrzahl elektrischer Leiter (140A-E) verbunden ist und/oder das redundant verbundene Kontaktelement (114A-C) an mindestens zwei voneinander beabstandeten Kontaktbereichen (114A.1,2, 114B.1,2, 114C.1,2) fest und elektrisch leitend mit je einem anderen der Mehrzahl elektrischer Leiter (140A-H) verbunden ist.

## Patentansprüche

1. Steckverbindungseinrichtung (100) zum Herstellen einer lösbaren elektrischen Verbindung zwischen zwei Komponenten eines mechanischen Herzunterstützungssystems, umfassend:
ein Steckverbindungselement (110), und
eine Mehrzahl elektrischer Leiter (140A-E); wobei
das Steckverbindungselement (110) aufweist:
einen länglich ausgebildeten Verbindungsabschnitt (112) zum Herstellen der lösbaren elektrischen Verbindung, und
eine Mehrzahl von voneinander isolierten elektrischen Kontaktelementen (114A-E), die entlang des Verbindungsabschnitts angeordnet sind; wobei
mindestens eines der Mehrzahl der elektrischen Kontaktelemente (114A-H) als redundant verbundenes Kontaktelement (114A-C) ausgeführt ist, wobei
das redundant verbundene Kontaktelement (114A-C) an mindestens zwei voneinander beabstandeten Kontaktbereichen (114A.1,2; 114B.1,2; 114C.1,2) fest und elektrisch leitend mit demselben der Mehrzahl elektrischer Leiter (140A-E) verbunden ist und/oder
das redundant verbundene Kontaktelement (114A-C) an mindestens zwei voneinander beabstandeten Kontaktbereichen (114A.1,2, 114B.1,2, 114C.1,2) fest und elektrisch leitend mit je einem anderen der Mehrzahl elektrischer Leiter (140A-H) verbunden ist.

2. Steckverbindungseinrichtung (100) nach Anspruch 1, wobei
jede der Kontaktelemente (114A) eine erste Oberfläche (114A.3) zur Herstellung der lösbaren elektrischen Verbindung und eine davon verschiedene zweite Oberfläche (114A.4) aufweist, und
die mindestens zwei voneinander beabstandeten Kontaktbereiche (114A.1,2) auf der zweiten Oberfläche angeordnet sind (114A.4).

3. Steckverbindungseinrichtung (100) nach einem der vorhergehenden Ansprüche, wobei entlang des Verbindungsabschnitts (112), vorzugsweise zwischen den elektrischen Kontaktelementen (114A-E), ein oder mehrere Dichtelemente (550), vorzugsweise eine oder mehrere Dichtlippen, angeordnet sind.

4. Steckverbindungseinrichtung (100) nach einem der vorhergehenden Ansprüche, wobei die Steckverbindungseinrichtung (100) ein Kabel (140) umfasst und die Mehrzahl der elektrischen Leiter (140A-E) Adern des Kabels (140) sind.

5. Steckverbindungseinrichtung (100) nach einem der vorhergehenden Ansprüche, wobei es sich bei dem Steckverbindungselement (110) um ein männliches Stechverbindungselement handelt.

6. Steckverbindungseinrichtung (300) nach einem der Ansprüche 1 bis 4, wobei es sich bei dem Steckverbindungselement (310) um ein weibliches Steckverbindungselement handelt.

7. Steckverbindungseinrichtung (300) nach Anspruch 6, wobei die Kontaktelemente (314A) jeweils als Federkontakte, vorzugsweise als Ringfederkontakte, ausgebildet sind.

8. Steckverbindungseinrichtung (100) nach einem der vorhergehenden Ansprüche, wobei
die Kontaktelemente (114A-E) jeweils den Verbindungsabschnitt (112) zumindest teilweise, vorzugsweise vollständig, umlaufen, und/oder
die Kontaktbereiche (114A.1,2) des redundant verbundenen Kontaktelementes (114A) entlang eines Umfangs des Kontaktelements (114A) verteilt angeordnet sind und um mindestens 30°, vorzugsweise mindestens 90°, 120° oder 180°, voneinander beabstandet sind.

9. Steckverbindungseinrichtung (100) nach einem der vorhergehenden Ansprüche, wobei die Steckverbindungseinrichtung (100) zumindest teilweise implantierbar ist.

10. Steckverbindungssystem (406) umfassend:
eine erste Steckverbindungseinrichtung (100) nach einem der vorhergehenden Ansprüche, wobei das Steckverbindungselement (110) der ersten Steckverbindungseinrichtung (100) ein erstes Steckverbindungselement ist, und
ein zweites Steckverbindungselement (310), wobei das zweite Steckverbindungselement (310) aufweist:
einen länglich ausgebildeten Verbindungsabschnitt (312) zum Herstellen der lösbaren elektrischen Verbindung,
eine Mehrzahl von voneinander isolierten elektrischen Kontaktelementen (314A-C), die entlang des Verbindungsabschnitts (312) angeordnet sind; wobei
das erste Steckverbindungselement (110) und das zweite Steckverbindungselement (310) zum lösbaren Herstellen der elektrischen Verbindung ineinander einführbar sind, wodurch ein paarweiser elektrischer Kontakt zwischen den Kontaktelementen des ersten Steckverbindungselements (110) und den Kontaktelementen des zweiten Steckverbindungselement (310) herstellbar ist.

11. Steckverbindungssystem (406) umfassend eine zweite Steckverbindungseinrichtung (300) nach einem der Ansprüche 1 bis 9, wobei deren Steckverbindungselement (310) das zweite Steckverbindungselement ausgebildet.

12. Mechanisches Herzunterstützungssystem (400) umfassend:
eine erste Komponente (402),
eine zweite Komponente (404), und
ein Steckverbindungssystem (406) nach Anspruch 10 oder 11, wobei eine lösbare elektrische Verbindung zwischen der ersten Komponente (402) und der zweiten Komponente (404) mittels des Steckverbindungssystems (406) herstellbar ist.

13. Männliches Steckverbindungselement (500) zum Herstellen einer lösbaren elektrischen Verbindung zwischen zwei Komponenten eines mechanischen Herzunterstützungssystems, umfassend:
einen länglich ausgebildeten Verbindungsabschnitt (112) zum Herstellen der lösbaren elektrischen Verbindung, und
eine Mehrzahl von voneinander isolierten elektrischen Kontaktelementen (514A-C), die entlang des Verbindungsabschnitts (112) angeordnet sind; wobei
entlang des Verbindungsabschnitts (112), vorzugsweise zwischen den elektrischen Kontaktelementen (514A-C), ein oder mehrere Dichtelemente (550), vorzugsweise eine oder mehrere Dichtlippen, angeordnet sind.

14. Steckverbindungssystem, umfassend:
ein männliches Steckverbindungselement (500) nach Anspruch 13, und
ein weibliches Steckverbindungselement, wobei das weinbliche Steckverbindungselement aufweist:
einen länglich ausgebildeten Verbindungsabschnitt zum Herstellen der lösbaren elektrischen Verbindung,
eine Mehrzahl von voneinander isolierten elektrischen Kontaktelementen, die entlang des Verbindungsabschnitts angeordnet sind; wobei
der Verbindungsabschnitt des männlichen Steckverbindungselements (500) zum lösbaren Herstellen der elektrischen Verbindung in den Verbindungsabschnitt des weiblichen Steckverbindungselements einführbar ist, wodurch ein paarweiser elektrischer Kontakt zwischen den Kontaktelementen (514A-C) des männlichen Steckverbindungselements (500) und den Kontaktelementen des weiblichen Steckverbindungselements herstellbar ist.
